(19) Europäisches Patentamt / European Patent Office / Office européen des brevets

(11) EP 1 383 804 B1

(12) **EUROPEAN PATENT SPECIFICATION**

(45) Date of publication and mention of the grant of the patent:
**11.07.2007 Bulletin 2007/28**

(21) Application number: **02704028.6**

(22) Date of filing: **14.03.2002**

(51) Int Cl.:
***C08B 11/14*** *(2006.01)* ***C08B 11/193*** *(2006.01)*
***C08L 1/28*** *(2006.01)*

(86) International application number:
**PCT/SE2002/000466**

(87) International publication number:
**WO 2002/079259 (10.10.2002 Gazette 2002/41)**

(54) **USE OF A QUATERNARY AMMONIUM ALKYL HYDROXYETHYL CELLULOSE ETHER AS A CONDITIONER FOR HAIR AND SKIN**

VERWENDUNG EINES QUATERNÄRE AMMONIUMGRUPPEN ENTHALTENDEN ALKYLHYDROXYETHYLCELLULOSEETHERS ALS HAAR- UND HAUT-CONDITIONER

UTILISATION D'UN ETHER DE CELLULOSE ALKYLE HYDROXYETHYLE A BASE D'AMMONIUM QUATERNAIRE EN TANT QUE CONDITIONNEUR POUR LES CHEVEUX ET POUR LA PEAU

(84) Designated Contracting States:
**AT BE CH CY DE DK ES FI FR GB GR IE IT LI LU MC NL PT SE TR**

(30) Priority: **29.03.2001 SE 0101108**

(43) Date of publication of application:
**28.01.2004 Bulletin 2004/05**

(73) Proprietor: **Akzo Nobel NV**
**6800 SB Arnhem (NL)**

(72) Inventors:
- **BUSK, Asa**
  **S-444 30 Stenungsund (SE)**
- **VAN DER HORST, P., M.**
  **NL-6843 XN Arnhem (NL)**
- **PLATE, Holger**
  **D-52379 Langerwehe (DE)**
- **TANG, Yonghua**
  **Suffern, NY 10901 (US)**

(74) Representative: **Alferink, Petrus J.T. et al**
**Akzo Nobel N.V.**
**Intellectual Property Dept.**
**P.O. Box 9300**
**6800 SB Arnhem (NL)**

(56) References cited:
**EP-A1- 0 956 850**
**EP-A2- 0 287 105**
**WO-A1-00/06656**
**WO-A1-01/08644**
**US-A- 3 472 840**
**US-A- 3 816 616**
**US-A- 3 876 760**
**US-A- 4 650 863**

EP 1 383 804 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

The present invention relates to the use of a certain quaternary ammonium cellulose ether derivative, which has improved conditioning properties as compared to prior known derivatives of this type, as a hair and skin conditioner.

Cationic cellulose derivatives containing 2-hydroxypropyl trialkyl ammonium chloride groups are widely used as conditioning agents in hair care and skin care products and a number of publications deal with this subject, see for example the review by Bernard Idson, Cosmet. Sci. Technol. Ser. (1999), 21, 251-279. The conditioning effect is obtained because the positively charged ammonium groups of the cationic cellulose derivatives are able to bind to anionic sites that are present on natural keratins, such as hair and skin. The hair thereby becomes easier to untangle when wet-combing, and when the hair is dry it will be more smooth, shiny and manageable. Both the combing forces and flyaway of the hair will be reduced. For skin, the cationic cellulose derivatives impart smooth feel, emolliency and skin protection.

The ability to act as a conditioner depends on how much of the substance is deposited onto the surfaces of the hair or skin, and also on how much of it is remaining after rinsing. The resistance to rinse off is called substantivity, and a good conditioner ought to have a large substantivity. However, too much substantivity might be negative, since there must not be a build-up of the conditioner on the hair on repeated use. The build-up will cause the hair to look lank, as well as to feel stiff and heavy.

The production of cationic cellulose ethers of the above-mentioned type has been described for example in US 3472840. They are obtained by reacting a cellulosic material with a 2,3-epoxypropyltrialkyl ammonium chloride or with a 3-chloro-2-hydroxypropyltrialkyl ammonium chloride in the presence of aqueous alkali hydroxide. The starting cellulosic material may be unsubstituted cellulose or a cellulose ether derivative, such as hydroxyethyl cellulose, hydroxypropyl cellulose or methyl cellulose.

Hydrophobically modified cellulose ethers, where the hydrophobic group has at least 10 carbon atoms, and containing a quaternary ammonium salt group, have been described in WO 00/06656 as rheology modifiers for waterborne coatings.

The cationic cellulose derivatives most widely used commercially as conditioners are the cationic hydroxyethyl cellulose (HEC) derivatives known as polyquaternium-10, which contain 2-hydroxy-3-(trimethylammonio)propyl ether groups. A number of publications are describing different compositions containing cationic cellulose derivatives in general, most often exemplified by the use of polyquaternium-10 derivatives. An early patent publication describing a shampoo-cream rinse composition is US 3816616, where it is disclosed that cationic cellulose derivatives, exemplified by cationic HEC, could be used in combination with an anionic surfactant. The disclosed composition is used for cleaning the hair as well as for conditioning it to improve the wet combing and general appearance.

Cellulose ethers are normally described by stating the number of alkyl or hydroxyalkyl substituents present on an average per anhydroglucose unit. The number of alkyl groups is given as the degree of substitution, DS, and the number of hydroxyalkyl groups as the molar substitution, MS. DS could maximally be 3, but MS could be larger since several oxyalkylene units may be added to the same position by further reaction of the hydroxyl group with more epoxide.

Now it has surprisingly been found that cationic cellulose derivatives based on a quaternary ammonium hydroxyethyl cellulose ether that comprises an ether substituent containing an alkyl group with 2-4 carbon atoms, preferably 2 carbon atoms, having a $DS_{alkyl}$ of 0.1-2.5, preferably 0.2-2.0 and most preferably 0.3-1.5, exhibit excellent properties as a hair and skin conditioner. For example, the quaternary ammonium hydroxyethyl cellulose ether exhibits an increased substantivity to hair, without showing any build-up after repetitive shampooing. Hair that has been treated with the above-mentioned cellulose ether also show an improved wet combability as compared to hair that has been treated with prior art cationic hydroxyethyl cellulose ethers. The $DS_{alkyl}$ is adapted to the hydrophobicity of the alkyl groups, which means that DS is normally lower for the more hydrophobic groups propyl and butyl than it is for ethyl. Suitably $DS_{ethyl}$ is 0.3-2.5, preferably 0.5-2.0 and most preferably 0.7-1.5.

The $MS_{hydroxyethyl}$ of the cellulose ethers of the present invention may vary within wide limits but is normally within a range of 0.4-4.0, suitably 1.0-3.0 and preferably 1.5-2.8. The $MS_{quaternary\ ammonium}$ is typically 0.02-2.0, preferably 0.04-1.0 and most preferably 0.05-0.5. The quaternary cellulose ether may also be substituted by longer hydrophobic groups, such as groups containing a hydrocarbon group derived from a glycidyl ether or a chloroglyceryl ether of a C5 to C22, preferably C10 to C18, alcohol or alkyl phenol that is preferably ethoxylated, or from a C5 to C22, preferably C10 to C18, alkyl halide. If so substituted, the product typically has a $DS_{hydrophobe}$ of 0.001-0.2, preferably 0.003-0.1 and most preferably 0.005-0.02. Examples of such longer hydrophobic groups can be found in US 4 228 277, EP-A-390 240, EP-A-426 086 and WO 00/08058. In addition to the alkyl groups with 2-4 carbon atoms, the cationic cellulose ether may also contain other alkyl groups, such as methyl groups. The methyl groups may for example be present in an amount yielding a $DS_{methyl}$ of 0.2-1.2.

The cellulose ether has a maximum of three positions that may be substituted, and the substituents may have the general formula

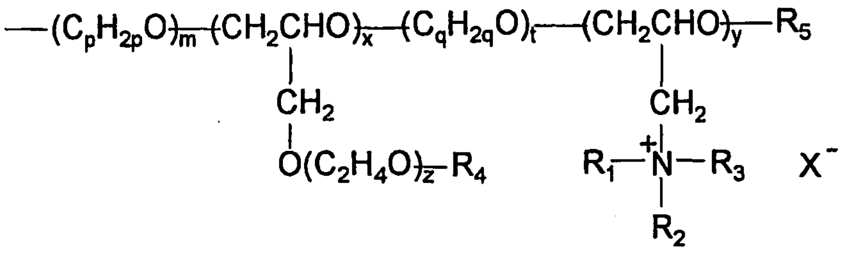

(I)

where p and q independently is 2 or 3, m is a number between 0-5, x is a number between 0-2, t is a number between 0-5, y is a number between 0-2, z is a number between 0-10, $R_1$, $R_2$ and $R_3$ independently of each other is an alkyl group with 1-22 carbon atoms, preferably 1-4 carbon atoms, $R_4$ is a hydrocarbon group with 5-22 carbon atoms, preferably 10-18 carbon atoms, $R_5$ is an alkyl group with 1-4 carbon atoms or H and $X^-$ is an anion, such as halide.

The quaternary ammonium cellulose ether of the invention may advantageously be used as a component in a conditioning shampoo, including 2-in-1 conditioning shampoos, in a hair conditioner, in a body wash or in bar soaps. A suitable formulation for a conditioning shampoo or body wash contains

1) a nonoinic, an anionic or an amphoteric surfactant or a blend thereof in a total amount of 5 to 30%, preferably 10 to 20%
2) 0.2 to 5% thickener and
3) 0.05 to 2%, preferably 0.1 to 1% and most preferably 0.2 to 0.5%, of a quaternary ammonium alkyl hydroxyethyl cellulose ether according to the invention, the composition having a pH between 5.0 to 7.0.

Suitable examples of nonionic surfactants are alkoxylated alcohols, alkyl polyglycosides, alkoxylated sorbitan esters, alkoxylated monoethanolamides, alkoxylated fatty acids and alkoxylated glycerides. The alkoxylates could contain a hydrophobic alkyl or acyl group with 8-22 carbon atoms, and the alkyleneoxy groups could be ethyleneoxy or propyleneoxy groups and the number of these groups could be between 2-15, preferably 3-10.

Suitable examples of anionic surfactants are alkyl sulphates, alkylaryl sulphates, alkyl ether sulphates, alkyl and alkylaryl sulphonates, olefinsulphonates, secondary alkyl sulphonates, sodium acyl isethionates, monoalkyl sulphosuccinates, acyl-N-alkyltaurates and protein-fatty acids condensates.

Suitable examples of amphoteric surfactants are N-alkyl betaines, N-alkyl glycinates, N-alkyl aminopropionates, N-alkyl iminodipropionates or alkyl imidazolines. Especially suitable examples are cocoamidopropyl betaine, cocodimethyl betaine, cocoamphocarboxy glycinate, cocoamphocarboxy propionates and coco or oleyl polyamino carboxylates.

The thickener could be an inorganic salt, such as sodium chloride or ammonium chloride; a cellulose ether, e.g. ethyl hydroxyethyl cellulose; or a synthetic polymer, such as polyacrylic acid derivatives, polyalkylene glycols and di- or polyurethanes of polyethoxylated compounds.

In addition the composition may also contain a skin compatible pH-adjustment agent, perfume oil, preservatives, opacifiers, pearlescent agents, dyes, humectants and refatting agents. The 2-in-1 shampoos most often also contain silicons, such as dimethicones, or silicone derivatives, e.g. quaternium 80, as additional conditioning agents. The conditioning shampoo and the body wash may also contain emollients and active ingredients, such as vitamins.

A suitable formulation for a hair conditioner contains

1) 0.05 to 2%, preferably 0.1 to 1% and most preferably 0.2 to 0.5%, of a quaternary ammonium alkyl hydroxyethyl cellulose ether according to the invention
2) 0.5 to 5%, preferably 1 to 4%, of a long chain fatty alcohol
3) a skin compatible acid in an amount sufficient to obtain a pH between 2.5 to 5.5, preferably 3-5 and most preferably around 3.5

The long chain fatty alcohol could contain 12 to 22 carbon atoms, preferably 16-18 carbon atoms.

The acid could for example be citric, lactic, tartaric, adipic or phosphoric acid or their salts.

The composition could also contain a thickener, for example a cellulose based thickener such as ethyl hydroxyethyl cellulose.

Another optional ingredient is a quaternary ammonium surfactant, such as mono- di- or trialkyl quats and mono- di- and triacyl ester quats. The quaternary compounds may also be ethoxylated.

Other ingredients that may be added are emulsifiers; oils such as silicon oils, triglycerides or mineral oil; dyes,

humectants, polyols, vitamins and a hydrophobic ester containing either a long chain fatty acid or a long chain fatty alcohol.

Still another embodiment of the invention is a bar soap containing a cationic cellulose derivative of the present invention. The bar soap could either be of the combi bar type, which includes natural soap as well as synthetic surfactants, or a syndet bar, which only includes synthetic surfactants. In addition, it could also be a bar soap that only includes natural soap.

A suitable solid composition for a bar soap contains

1) an anionic surfactant, which is a synthetic anionic surfactant and/or a soap, or an amphoteric surfactant or a blend thereof in a total amount of 40-90%
2) 10 to 60% of a plasticiser
3) 0.05 to 2%, preferably 0.1 to 1% and most preferably 0.2 to 0.5%, of a quaternary ammonium alkyl hydroxyethyl cellulose ether according to the invention.

Suitable examples of anionic surfactants are alkyl sulphates, alkylaryl sulphates, alkyl ether sulphates, alkyl and alkylaryl sulphonates, olefinsulphonates, secondary alkyl sulphonates, sodium acyl isethionates, monoalkyl sulphosuccinates, acyl-N-alkyltaurates, protein-fatty acids condensates, and fatty acid soaps.

Suitable examples of amphoteric surfactants are N-alkyl betaines, N-alkyl glycinates, N-alkyl aminopropionates, N-alkyl iminodipropionates or alkyl imidazolines. Especially suitable examples are cocoamidopropyl betaine, cocodimethyl betaine, cocoamphocarboxy glycinate, cocoamphocarboxy propionates and coco or oleyl polyamino carboxylates.

The plasticiser could for example be a fatty acid or paraffin waxes or mixtures thereof.

Other ingredients that may be added are humectants, perfume oils, dyes, disinfectants, complexing agents and titanium dioxide.

The quaternary ammonium cellulose ethers of the present invention, for example 2-hydroxy-3-(trimethylammonio)propyl ethyl hydroxyethyl cellulose, are soluble in ethanol/water mixtures containing a high proportion of ethanol. This property makes these compounds suitable for use in alcoholic hair styling gels as well as in alcohol based hair sprays. The cellulose ethers, as well as the formulations thereof, can also find use in paper processing, detergents, fabric care, hard surface cleaning, hair colouring, plasters, coatings formulas, mining agents, asphalt emulsification, oilfield rheology control, pharmaceutical applications such as coating agents for solid products or viscosifiers, viscose processing, and as a transfer agent of substances such as preservatives.

The quaternary ammonium cellulose ether of the present invention may be produced by general methods described in for example US 3472840. The order of the different steps of the process is optional, but preferably the quaternary ammonium compound, e.g. a cationic epoxide such as glycidyl trimethylammonium chloride, or the corresponding halohydrin, e.g. 3-chloro-2-hydroxypropyl trimethylammonium chloride, is added to the prefabricated hydroxyethyl cellulose ether comprising an alkyl group with 2-4 carbon atoms, the alkyl group having a $DS_{alkyl}$ of 0.1-2.5. The prefabricated cellulose ether is made by adding an epoxide, such as ethylene oxide or propylene oxide, and an alkyl halide, such as ethyl, propyl or butyl chloride, to alkali cellulose. If this route is followed, the prefabricated cellulose ether that is used as starting material preferably has a turbidity point (flocculation temperature) of below 100°C and most preferably from 25°C to 85°C. The cellulose ether may also be hydrophobically modified, for example with aliphatic groups containing 6-18 carbon atoms. Such modification may be obtained by reacting the cellulose ether with an epoxide obtained from a fatty alcohol or an ethoxylated fatty alcohol via the epichlorohydrin route, or from a long-chain alkyl halide. Suitable nonionic cellulose derivatives that may be used as starting materials for the cationic cellulose derivatives of the present invention are the ethyl hydroxyethyl cellulose, propyl hydroxyethyl cellulose or butyl hydroxyethyl cellulose, or the hydrophobically modified derivatives thereof. The above-mentioned nonionic cellulose derivatives may also be further reacted with other alkyl halides, for example with methyl chloride. The preferred starting material is ethyl hydroxyethyl cellulose (EHEC) or hydrophobically modified ethyl hydroxyethyl cellulose. The most preferred EHEC materials include, but are not limited to, water soluble or swellable EHEC derivatives and/or alcohol soluble (e.g. ethanol) EHEC derivatives. It should also be understood that substituted EHEC derivatives (e.g. methylated, carboxymethylated or hydroxypropylated EHEC) are within the scope and meaning of the EHEC starting materials.

The EHEC or EHEC derivative starting material is provided with quaternary ammonium groups through various methods known to those of ordinary skill in the art. To prepare cationic EHEC derivatives one can start either with cellulose or with cellulose that has already been reacted with ethylene oxide and ethyl chloride and optionally with other non-ionic and/or anionic alkylating agents. To provide the EHEC or EHEC derivative with the cationic group an etherification reaction in the presence of caustic soda may be performed with either 3-chloro-2-hydroxypropyl trialkylammonium chloride or a glycidyl trialkylammonium chloride. Preferably all three alkyl groups are methyl groups, as in the compounds displayed below.

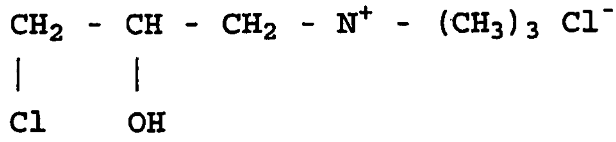

3-Chloro-2-hydroxypropyl trimethylammonium chloride (CHPTAC)

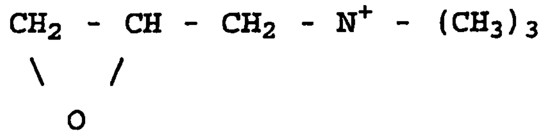

Glycidyl trimethylammonium chloride (GTAC)

When using CHPTAC one equivalent alkali metal hydroxide will be consumed to form the epoxide, which is the reagent that takes part in the etherification reaction, and one equivalent of NaCl will be produced. The reaction of GTAC with EHEC needs only a catalytic amount of alkali metal hydroxide. In case the etherification is performed by the sequence of first reacting the alkali cellulose with ethylene oxide and ethyl chloride, after which the etherification with CHPTAC or GTAC is performed without a purification of the EHEC, some of the CHPTAC or GTAC might also react with the by-products resulting from the etherification with ethylene oxide and ethyl chloride. This is also the case when the etherification with ethylene oxide, ethyl chloride and CHPTAC or GTAC is performed at the same time. Other cationic EHEC derivatives can be derived in the same way as the 2-hydroxypropyl trimethylammonium EHEC derivative is prepared and general processes to do so are known in the art.

Production of water swellable and water soluble polysaccharide ethers in general is done by suspending the polysaccharide in a diluent. Suitable diluents include but are not limited to ethanol, isopropyl alcohol, tert-butyl alcohol, acetone, water, methylethyl ketone and mixtures thereof. The reactions may be conducted in a relatively large amount of diluent or with a minimal amount of diluent as desired, i.e., using either a so-called slurry or a so-called dry process, respectively.

Typically, the polysaccharide is reacted with an alkali metal hydroxide to prepare an alkali metal polysaccharide. The amount of alkali metal hydroxide per saccharide repeating unit may vary, depending on the type and amount of alkylating agents used. Typically, a molar ratio of hydroxide to saccharide repeating unit of between 0.001 and 5 is used. If desired, during the alkylation additional alkali metal hydroxide can be added, or excess of the alkali metal hydroxide can be neutralised. To prevent uncontrolled degradation of the alkalised polysaccharide polymer, it is preferred to exclude oxygen from the reaction vessel during the alkalisation and alkylation.

For the production of a cationic polysaccharide, the alkali metal polysaccharide may first be reacted with the non-ionic alkylating reagents, e.g. ethylene oxide and ethyl chloride, followed by the reaction with the cationic alkylating reagent, e.g. CHPTAC or GTAC, at a suitable temperature and for a time sufficient to provide the desired level of substitution. Alternatively, the cationic alkylating reagent may be added first, after which the other alkylating agents, e.g. non-ionic alkylating reagents are allowed to react, or the alkali metal polysaccharide may be simultaneously reacted with the different alkylating reagents. A further alternative reaction path is to purify the non-ionically modified polysaccharide before the cationic alkylating reagent is added. This generally increases the reaction selectivity and/or yield of the cationic alkylating reagent.

In another embodiment of the present invention also other non-ionic and/or anionic alkylating reagents are incorporated in the reaction step, either added before, after or together with the ethylene oxide, ethyl chloride and cationic alkylating reagents. Also here the cationisation may be performed after the already alkylated nonionic and/or anionic polysaccharide has been purified. Suitable examples of these other alkylating reagents are hydrophobic epoxides or halides containing a hydrocarbon group with five or more carbon atoms, such as dodecanol+4EO-glycidyl ether, nonylphenol+4EO-glycidyl ether and dodecyl bromide; methyl chloride, propylene oxide, butylene oxide or sodium chloroacetate.

In the context of the present invention the quaternary ammonium modified EHEC and its derivatives may be applied either as purified or as unpurified materials, preferably as purified. The by-products have been removed from the purified materials e.g. by extraction with hot salt water or with an alcohol/water mixture.

The quaternary ammonium hydroxyethyl cellulose ethers described above and a process for their production are already partly disclosed in the earlier mentioned publication WO 00/06656 for use as rheology modifiers for waterborne coatings. The cellulose ethers disclosed in said publication contain at least one hydrophobic group selected from the group consisting of aryl, alkyl, alkenyl, aralkyl and mixtures thereof, and having at least 10 carbon atoms. In addition to said hydrophobic group the cellulose ethers also contain at least one quaternary ammonium salt group. However, the

presence of these hydrophobic groups is not essential for the present invention. Therefore, the invention also relates to the quaternary ammonium hydroxyethyl cellulose ethers per se and a process for their production, with the proviso that the cellulose ethers do not contain any hydrophobic group, selected from the group consisting of aryl, alkyl, alkenyl, aralkyl and mixtures thereof, and having at least 10 carbon atoms, the hydrophobic group not being a part of any quaternary ammonium group.

The following examples are illustrative of the invention, and should not be considered as limiting thereof.

**Example 1**

2-Hydroxy-3-(trimethylammonio)propyl ethyl hydroxyethyl cellulose ether chloride (Q-EHEC) was produced by the so-called dry process, using a Drais reactor equipped with a plowshare mixer. The starting material was ethyl hydroxyethyl cellulose ether (EHEC) with a purity of about 95% on dry basis. The EHEC used had an average $MS_{EO}$ of 2.1 and an average $DS_{Ethyl}$ of 0.9. ($MW_{sacch}$ = 280) The Brookfield viscosity at 2% concentration of the EHEC was 2200 $\pm$ 450 mpas with spindle/speed 3/12, which corresponds to an average degree of polymerisation of 650. This would yield an average molecular weight of around 180 000.

350 gram (1.12 mol) of the EHEC was added to a 2.5 1 Drais reactor. After the reactor had been closed 70 g of 87 wt% aqueous isopropyl alcohol and 70 g water was sprayed on the reaction mass while stirring. After the reactor content had been brought under a nitrogen atmosphere 49.3 g of a 50% aqueous NaOH solution was added to the reactor. After stirring for 15 min. 84.2 of a 69% aqueous solution of 3-chloro-2-hydroxypropyl trimethylammonium chloride was added to the reactor. Then the temperature of the reaction mass, which until now had been at room temperature, was raised to 65°C. The reaction mass was maintained at this temperature for two hours to complete the reaction, after which it was neutralised by addition of aqueous acetic acid. Then the reactor was unloaded.

The product was purified by first dispersing the material in hot salt water (15% NaCl; 90°C) after which the slurry was filtrated and further purified by washing with additional hot salt water till the quaternary ammonium by-products were removed to a level of < 0.2% by weight. This was checked by using Capillary Zone Electrophoresis analysis methods. The nitrogen content as determined by a Kjeldahl analysis of the purified (but still containing 3 wt% NaCl) Q-EHEC was 1.05 wt%, which corresponds to a $MS_{quaternary\ ammonium}$ of 0.24. This product is hereafter referred to as Q-EHEC A.

An analogous product was produced from an ethyl hydroxyethyl cellulose ether with a $DS_{ethyl}$ of 0.9, a $MS_{hydroxyethyl}$ of 2.1 and a Brookfield viscosity at 2% concentration of the EHEC of 300 $\pm$ 60 mPas with spindle/speed 1/12, which corresponds to an average degree of polymerisation of 300, and which would yield an average molecular weight of around 80 000. The nitrogen content of this product as determined by Kjeldahl analysis was 0.93, which corresponds to a $MS_{quaternary\ ammonium}$ of 0.21. This product is hereafter referred to as Q-EHEC B.

**Example 2**

The surface tension was determined according to du Nouy (DIN 53914) at 25°C for a 0.1% solution of Q-EHEC A, Q-EHEC B and two different commercially available 2-hydroxy-3-(trimethylammonio)propyl hydroxyethyl cellulose ether chlorides (Q-HEC) that were used as references.

| Product | Surface tension (mN/m) |
|---|---|
| Q-EHEC A | 49 |
| Q-EHEC B | 49 |
| Leogard GP[1] (Q-HEC) (reference) | 60 |
| Polymer JP 400[2] (Q-HEC) (reference) | 65 |
| Water (reference) | 72 |

[1]Leogard GP is a hydroxyethyl 2-hydroxy-3-(trimethylammonio) propyl cellulose ether chloride with a $MS_{hydroxyethyl}$ of 1.8 and a $MS_{quaternary\ ammonium}$ of 0.38

[2]Polymer JR 400 is a hydroxyethyl 2-hydroxy-3-(trimethylammonio) propyl cellulose ether chloride with a $MS_{hydroxyethyl}$ of 1.9 and a $MS_{quaternary\ ammonium}$ of 0.35

It is evident from the surface tension values that the Q-EHEC derivatives are more surface active than the Q-HEC

derivatives.

**Example 3**

The foaming behaviour of the two Q-EHEC products from Example 1 was compared to the same references as in Example 1. The foam was measured as mm foam produced in a 500 ml measuring cylinder with a 49 mm inner diameter from 200 ml 0.05% solution of the quaternary ammonium cellulose ethers in distilled and demineralised water, when the cylinder is turned around 40 times within one minute. The test was performed at room temperature, and the foam height was registered directly and after 5 minutes.

| Product | Foam height (mm) after 0 min | Foam height (mm) after 5 min |
|---|---|---|
| Q-EHEC A | 74 | 72 |
| Q-EHEC B | 72 | 61 |
| Leogard GP (reference) | 20 | 5 |
| Polymer JR 400 (reference) | 14 | 0 |

From the foam height values it is obvious that the Q-EHEC derivatives are better foamers than the Q-HEC derivatives.

**Example 4**

The substantivity of the cationic cellulose derivatives was investigated by ESCA (Electron Spectroscopy for Chemical Analysis). There is an elaborated description of this technique in e.g. the electronic version (year 2000) of Ullmann's Encyclopedia of Industrial Chemistry, Chapter on Surface Analysis. A Q-HEC derivative was used as a reference for the ESCA measurements.

Description of method:

A 1% solution of cationic cellulose derivative was prepared in distilled water.

A normal brown hair was first washed with a 10% sodium lauryl sulphate (SLS) solution for one minute. The hair was dipped into 5g of the SLS solution and washed by rubbing the hair between the fingers. After washing the hair was thoroughly rinsed with distilled water. The hair tress sample was then split in two, one part for the blank, the other for the treatment. The portion for the blank was set aside to dry. For all blanks, the $N^+$ value was zero.

The washed hair was dipped into 2.5 g of the cationic cellulose derivative solution and rubbed between the fingers for one minute. The hair was thoroughly rinsed and allowed to air-dry.

The ESCA measurement was performed as follows:

XPS spectra were collected at room temperature with a Physical Electronics 5600ci electron spectrometer. Binding energy scale calibration was performed as described in the E-42 ASTM-902 document.

Samples for every analysis were taken from as near the mid-point along the length of the hair tress as could be reproducibly achieved. Hair fibres were mounted between two, three hole, sample cover masks, (PHI part # 612448) to hold the fibres co-planar and parallel.

Instrument control, data collection and data analysis were all performed with Physical Electronics PC-ACCESS software running on a HP Vectra Pentium PC. Quantitation calculations were based on measured peak areas and empirical instrumental sensitivity factors within the above mentioned software. Curve fitting analysis employed an integral background and mixed Gaussian - Lorentzian peak shape. The XPS quantities are expressed as atomic percent.

The amounts of $N^+$ in the cationic cellulose polymers were determined by Kjeldahl analysis.

| Product | $N^+$ content (%) in the polymer (by Kjeldahl analysis) | Atomic % $N^+$ on hair fibre (by ESCA) |
|---|---|---|
| Q-EHEC A | 1.05 | 0.5 |
| Q-EHEC B | 0.93 | 0.6 |

(continued)

| Product | $N^+$ content (%) in the polymer (by Kjeldahl analysis) | Atomic % $N^+$ on hair fibre (by ESCA) |
|---|---|---|
| Polymer JR 400 (Q-HEC) (reference) | 1.75 | 0.55 |

Although the amount of positively charged nitrogen adsorbed on the hair fibre is equal for the Q-EHEC derivatives and the Q-HEC reference, the total amount of polymer adsorbed must be almost twice as large for the Q-EHEC derivatives since the $N^+$ content in the Q-EHEC polymer is only about half of the amount in Q-HEC.

**Example 5**

The build-up on hair fibres was measured by means of microfluorometry. This method involves incorporation of an appropriate fluorochrome (labelling agent) into the formulation, and provides information about the thickness of surface deposition of the conditioning polymers. This technique can also be used to study the build-up effect due to multiple treatments.

Hair Treatment Procedure:

1. Each hair bundle was treated with shampoo base solution (10% sodium lauryl ethersulphate and 3% cocoamidopropyl betaine) for one minute, followed by rinsing and drying.
2. 10 to 15 hair fibres were removed from the hair bundle for testing the shampoo control by microfluorometry.
3. Above-mentioned 10 to 15 hair fibres were re-placed into the bundle, which was washed 2 minutes with the conditioning shampoo solution (10% sodium lauryl ethersulphate, 3% cocoamidopropyl betaine, 0.3% Q-EHEC and 0.03% CIFB-113 (4,4'-bis[(anilino-6-[bis(2-hydroxyethyl)amino]-s-triazine-2-yl)amino]-2,2'-stilbenedisulfonate; label)). Then the fibres were rinsed with warm water and dried.

Quantification of the extent of the build-up, or lack thereof, was achieved by carrying out microfluorometric scans along the length of the same hair fibres before and after single and multiple applications of the labelled shampoo-based conditioner solutions.

In the table below is collected the average fluorescence intensity data for an untreated, a single-treated and a multiple-treated sample consisting of ten hair fibres. The fluorescence intensity emissions were measured at $\lambda_{max}$ = 450 nm and are given as %-values.

| Product | Control (washed but not treated with conditioner | Single treatment | Multiple treatment | Adsorption (single treatment - control) |
|---|---|---|---|---|
| Q-EHEC A | 3.60 | 4.10 | 4.22 | 0.50 |
| Q-EHEC B | 3.78 | 4.04 | 4.00 | 0.26 |
| Polymer JR 400 | 4.15 | 4.30 | 4.38 | 0.15 |

The Q-EHEC derivatives did not show any conditioning polymer build-up on hair surface after multiple treatments of the respective shampoo-based conditioners, as is shown by the comparison between the single treated and the multiple treated samples. All scans yielded rather low and uniform fluorescence emission intensities, which indicates negligible build-up. From the single treatment data, both the Q-EHEC A and the Q-EHEC B derivatives showed the trend of slightly greater affinity to hair fibre than Polymer JR 400, which is in agreement with the ESCA measurements.

**Example 6**

The Diastron Wet Combability test, using the MTT 170 Diastron, was performed to measure the conditioning effectivity.

The measurements were performed on bleached hair tresses. All tresses were washed with 10% sodium lauryl

sulphate and thoroughly rinsed before use. Each bleached hair tress was combed through once to remove major tangles and then combed 5 times on the MTT 170 Dia-stron at 150 mm/min. The tress was immersed in water and gently squeezed out between two fingers before each comb through. The computer recorded the force (gram metric force, g) required to comb through the tresses, and the total combing work (Joule, J) was obtained by computing the area under the complete combing force curve.

After the untreated tresses had been combed to obtain the control value, the tresses were wet under flowing tap water at 40°C for 1 minute. Then 2 ml of cationic cellulose polymer-containing solution (10% sodium lauryl ethersulphate (2 moles of ethylene oxide), 3% cocoamidopropyl betaine and 0.3% conditioning polymer) was applied and gently lathered in for 1 minute, after which the tress was rinsed for 5 min in the warm flowing water. The tresses were then combed again as described previously. The data are expressed as the percent reduction in combing peak force or combing work of an individual hair tress as a result of treatment. Four replicates with different hair tresses were performed for each conditioner investigated.

| Product | Reduction of total combing work (%) | Reduction of peak combing force (%) |
|---|---|---|
| Q-EHEC A | 76 | 78 |
| Q-EHEC B | 69 | 69 |
| Polymer JR 400 (ref) | 58 | 62 |

From these tests it is clearly shown that both the reduction in total comb work as well as the reduction of the peak combing force is greater for the cationic cellulose conditioners of the invention than for the cationic hydroxyethyl cellulose.

**Example 7**

The adsorption of the conditioner on hair fibres was also investigated by wettability experiments.

Six fibers were used for each of the products and the results reported below will be the average of six measurements. All the fibers used were picked from a dark brown hair tress (De Meo Bros., New York) that had been washed in sodium lauryl sulfate solution (10% SLS, hair-to-SLS weight ratio 1:0.5, gentle rubbing with hand for one min, followed by thorough rinsing in deionised water for 15 min).

Each set of six fibres for a particular conditioner was then subjected to the following treatment. The fibres were dipped into a solution containing 10% SLES + 1% of the respective conditioner, and the solution was gently spreading along the length. The fibres were then rinsed with deionised water for 30 s and dried overnight. Wettability experiments were performed to calculate θ, which is the contact angle for the partially conditioner-covered fibre surface, by using the TRI Wetting Force Scanner based on the Wilhelmy principle. The fibres were then dried overnight again, then dipped in 0.5% of the pure conditioner in water, gently withdrawn, dried for a few minutes and dipped again. This was done 3 times to ensure total coverage of the fibre surface with the pure conditioner compound. Without washing, the fibres were then dried overnight and then the wettability measurement performed. This would yield $\theta_t$, the contact angle for the completely covered fibre surface.

After the washing step described above, the control samples were dipped in deionised water overnight to completely leach out any SLS left behind. They were then dried overnight to dry before the wettability measurements were performed. These measurements would yield $\theta_u$, the contact angle for the bare fibre surface.

By separately determining the contact angles of both the completely covered ($\theta_t$) and bare ($\theta_u$) fibre surfaces, it can be shown that for a partially covered surface, the fraction covered, $X_t$, is given by

$$X_t = (\mathrm{Cos}\ \theta - \mathrm{Cos}\ \theta_u)/(\mathrm{Cos}\ \theta_t - \mathrm{Cos}\ \theta_u)$$

where θ is the contact angle for the partially covered surface.

| Product | Fraction of the surface covered ($X_t$) |
|---|---|
| Q-EHEC B | 0.118 |
| Polymer JR 400 (Q-HEC) | 0.031 |

The results show a higher adsorption for hair treated with the Q-EHEC derivative than for the reference, which is in line with the ESCA and the microfluorometric measurements.

**Example 8**

A composition for a conditioning shampoo according to the invention is formulated as follows:

| Ingredients | Weight (%) |
|---|---|
| Elfacos T212[1] | 1.0 |
| Sympatens ALM-020[2] | 0.5 |
| Q-EHEC A[3] | 0.3 |
| Elfan NS242 (27% a.s.)[4] | 37 |
| Tego Betain L7 (30% a.s.)[5] | 10 |
| Euxyl 700[6] | 1.0 |
| Citric acid | Added to obtain pH 5 |
| Water | balance |

The surfactants, the conditioning polymer and the thickener are added to water at 40°C.
The mixture is stirred at this temperature until all the ingredients are dissolved. Then citric acid is added to adjust the pH, and finally the preservative is added below 30°C. The formulation obtained is a clear solution having a low shear Brookfield viscosity at 20°C of approximately 7000 mPas.
[1]Elfacos T212 = (Palmityl alcohol + 14 PO + 60 EO) hexyl dicarbamate (PPG-14 Palmeth-60 Hexyl Dicarbamate)
[2]Sympatens ALM-020= Lauryl alcohol + 2 EO (Laureth-2)
[3]Q-EHEC A = as defined in Example 1
[4]Elfan NS242 = Sodium lauryl ether (2EO) sulfate (Sodium Laureth Sulfate)
[5]Tego Betain L7= Cocoamidopropyl betaine
[6]Euxyl 700= mixture of phenoxyethanol, benzyl alcohol, potassium sorbate, tocopherol and water
The INCI-names are given in parenthesis when appropriate.

**Example 9**

A composition for a hair conditioner according to the invention is formulated as follows:

| Ingredient | Weight (%) |
|---|---|
| Elfacos CD 481[7] | 0.9 |
| Elfacos OW 100[8] | 0.5 |
| Lanette 16[9] | 2.5 |
| Kessco IPS[10] | 3.0 |
| Q-EHEC A[11] | 0.3 |
| Arquad 16-29 (29%)[12] | 1.7 |
| Citric acid | added to obtain pH 3.7 |
| Euxyl 700[13] | 1.0 |

(continued)

| Ingredient | Weight (%) |
|---|---|
| Water | balance |
| The first four ingredients are added to water at 75°C in the same order as they are displayed in the table. The mixture is stirred at this temperature until all the ingredients are dissolved. The temperature is lowered to 40°C and the Q-EHEC and Arquad are added. Then citric acid is added to adjust the pH, and finally the preservative is added below 30°C. The formulation obtained is a white stable emulsion having a low shear Brookfield viscosity at 20°C of approximately 22000 mPas.<br>[7]Elfacos CD 481 = Ethyl hydroxyethyl cellulose<br>[8]Elfacos OW 100 = Methylblocked polyethylene glycol (17EO)/Dodecyl glycol copolymer (Methoxy PEG-17/Dodecyl Glycol Copolymer)<br>[9]Lanette 16 = Cetyl alcohol<br>[10]kessco IPS = Isopropyl stearate<br>[11]Q-EHEC A = as defined in Example 1<br>[12]Arquad 16-29 = hexadecyltrimethyl ammonium chloride (Cetrimonium chloride)<br>[13]Euxyl 700 = mixture of phenoxyethanol, benzyl alcohol, potassium sorbate, tocopherol and water<br>The INCI-names are given in parenthesis when appropriate. | |

**Example 10**

A composition for a conditioning body wash according to the invention is formulated as follows:

| Ingredient | Weight (%) |
|---|---|
| Elfan AT 90 G (86%)[14] | 5.8 |
| Q-EHEC A[15] | 0.3 |
| Elfan NS242 (27%)[16] | 18.7 |
| Elfacos T212[17] | 1.0 |
| Sympatens ALM/020[18] | 0.5 |
| Tego Betain L7 (30%)[19] | 10.0 |
| Citric acid | added to obtain pH 5.3 |
| Euxyl 700[20] | 1.0 |

(continued)

| Ingredient | Weight (%) |
|---|---|
| Water | balance |
| The first six ingredients are added to water at 40°C in the same order as they are displayed in the table. The mixture is stirred at this temperature until all the ingredients are dissolved. Then citric acid is added to adjust the pH, and finally the preservative is added below 30°C. The formulation obtained is a clear solution having a low shear Brookfield viscosity at 20°C of approximately 4500 mPas.<br>[14]Elfan AT 90 G (86%) = Sodium cocoyl isethionate<br>[15]Q-EHEC A = as defined in Example 1<br>[16]Elfan NS242 (27%)= Sodium lauryl ether (2EO) sulfate (Sodium Laureth Sulfate)<br>[17] Elfacos T212= (Palmityl alcohol + 14 PO + 60 EO) hexyl dicarbamate (PPG-14 Palmeth-60 Hexyl Dicarbamate)<br>[18]Sympatens ALM/020= Lauryl alcohol + 2 EO (Laureth-2)<br>[19]Tego Betain L7 (30%)= Cocoamidopropyl betaine<br>[20]Euxyl 700 = mixture of phenoxyethanol, benzyl alcohol, potassium sorbate, tocopherol and water<br>The INCI-names are given in parenthesis when appropriate. | |

## Claims

1. Use of a quaternary ammonium hydroxyethyl cellulose ether, **characterised in that** it comprises an ether substituent containing an alkyl group with 2-4 carbon atoms having a $DS_{alkyl}$ of 0.1-2.5, as a hair or skin conditioner.

2. Use of a cellulose ether according to claim 1, **characterised in that** the alkyl group comprises an ethyl group having a $DS_{ethyl}$ of 0.3-2.5.

3. Use of a cellulose ether according to claims 1-2, **characterised in that** the cellulose ether has a $MS_{hydroxyethyl}$ of 0.4-4.0.

4. Use of a cellulose ether according to claims 1-3, **characterised in that** it has a $MS_{quaternary\ ammonium}$ of 0.02-2.0.

5. Use of a cellulose ether according to claims 1-4, **characterised in that** the quaternary ammonium group is a 2-hydroxypropyl trialkyl ammonium chloride group, where the alkyl groups contain 1-22 carbon atoms.

6. Use of a cellulose ether according to claims 1-5, **characterised in that** the quaternary ammonium group is a 2-hydroxypropyl trialkyl ammonium chloride group, where the alkyl groups contain 1-4 carbon atoms.

7. Use of a cellulose ether according to claims 1-6, **characterised in that** the quaternary ammonium group is a 2-hydroxypropyl trimethyl ammonium chloride group.

8. Use of a cellulose ether according to claims 1-7, **characterised in that** it comprises substituents of the formula

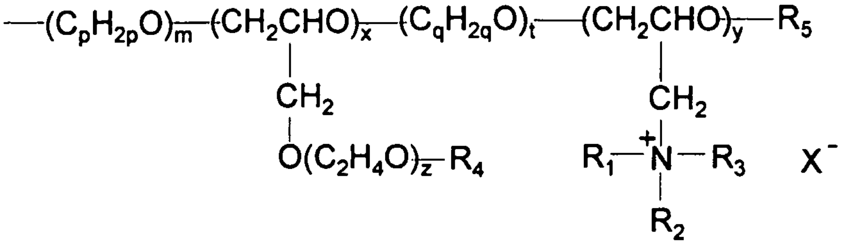

(I)

where p and q independently is 2 or 3, m is a number between 0-5, x is a number between 0-2, t is a number between 0-5, y is a number between 0-2, z is a number between 0-10, $R_1$, $R_2$ and $R_3$ independently of each other is an alkyl group with 1-22 carbon atoms, $R_4$ is a hydrocarbon group with 5-22 carbon atoms, $R_5$ is an alkyl group with 1-4 carbon atoms or H and $X^-$ is an anion.

**9.** Use of a cellulose ether according to claim 8, **characterised in that** x=0.

**10.** Use of a cellulose ether according to claim 8, **characterised in that** it has a MS of 0.001-0.2 for the group

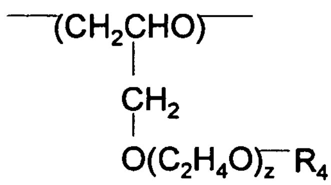

where $R_4$ and z have the same meaning as in formula (I).

**11.** Use of a cellulose ether according to claims 1-10, **characterised in that** $R_5$ partially is methyl.

**12.** Use of a cellulose ether according to claims 1-11, where $R_1$, $R_2$ and $R_3$ independently of each other is an alkyl group with 1-4 carbon atoms.

**13.** Use of a cellulose ether according to claims 1-12, **characterised in that** p and q are 2.

**14.** Use of a cellulose ether according to claims 1-13, **characterised in that** $R_1$, $R_2$ and $R_3$ are $-CH_3$.

**15.** A quaternary ammonium hydroxyethyl cellulose ether, **characterised in that** the cellulose ether has the structure as defined in claims 1-14, with the proviso that the cellulose ether does not contain any hydrophobic group, selected from the group consisting of aryl, alkyl, alkenyl, aralkyl and mixtures thereof, and having at least 10 carbon atoms, the hydrophobic group not being a part of any quaternary ammonium group.

**16.** A process for producing a quaternary ammonium alkyl hydroxyethyl cellulose ether according to claim 15, **characterised in that** the cellulose ether is produced by reacting a quaternary ammonium compound with a hydroxyethyl cellulose ether comprising an ether substituent containing an alkyl group with 2-4 carbon atoms and a $DS_{alkyl}$ of 0.1-2.5 in the presence of alkali.

**17.** A process according to claim 16 where the quaternary ammonium compound is a 2,3-epoxypropyltrialkyl ammonium chloride or a 3-chloro-2-hydroxypropyltrialkyl ammonium chloride, where the alkyl groups contain 1-22 carbon atoms.

**18.** A process according to claim 17 where the alkyl groups contain 1-4 carbon atoms.

**19.** A process according to claim 18 where the alkyl groups are methyl groups.

**20.** An aqueous hair or skin conditioning composition, **characterised in that** it contains

- a quaternary ammonium alkyl hydroxyethyl cellulose ether as defined in claim 1-15,
- a nonionic, an anionic or an amphoteric surfactant or a blend thereof, and
- a thickener.

**21.** An aqueous composition according to claim 20 to be used in a conditioning shampoo or body wash containing

1) the nonionic, an anionic or an amphoteric surfactant or a blend thereof in a total amount of 5 to 30%
2) 0.2 to 5% thickener, and
3) 0.05 to 2% of the quaternary ammonium alkyl hydroxyethyl cellulose ether,

the composition having a pH between 5.0 to 7.0.

**22.** An aqueous composition according to claim 20 to be used in a hair conditioner containing

1) 0.05 to 2% of the quaternary ammonium alkyl hydroxyethyl cellulose ether
2) 0.5 to 5% of a long chain fatty alcohol
3) a skin compatible acid in an amount sufficient to obtain a pH between 2.5 to 5.5.

**23.** A solid soap bar composition, **characterised in that** it contains a quaternary ammonium alkyl hydroxyethyl cellulose ether as defined in claim 1-15 and a syndet, natural or combi soap.

**24.** A solid soap bar composition according to claim 23 containing

1) an anionic surfactant, which is a synthetic anionic surfactant and/or a soap, or an amphoteric surfactant or a blend thereof in a total amount of 40-90%
2) 10 to 60% of a plasticiser
3) 0.05 to 2% of the quaternary ammonium alkyl hydroxyethyl cellulose ether.

## Patentansprüche

**1.** Verwendung eines quaternären Ammoniumhydroxyethylcelluloseethers, **dadurch gekennzeichnet, dass** er einen Ethersubstituenten umfasst, der eine Alkylgruppe mit 2 - 4 Kohlenstoffatomen mit einem $DS_{Alkyl}$ von 0,1 - 2,5 enthält, als Haar- oder Hautkonditionierer.

**2.** Verwendung eines Celluloseethers nach Anspruch 1, **dadurch gekennzeichnet, dass** die Alkylgruppe eine Ethylgruppe mit einem $DS_{Ethyl}$ von 0,3 - 2,5 umfasst.

**3.** Verwendung eines Celluloseethers nach den Ansprüchen 1-2, **dadurch gekennzeichnet, dass** der Celluloseether eine $MS_{Hydroxyethyl}$ von 0,4 - 4,0 hat.

**4.** Verwendung eines Celluloseethers nach den Ansprüchen 1-3, **dadurch gekennzeichnet, dass** er eine $MS_{quaternäres\ Ammonium}$ von 0,02 - 2,0 hat.

**5.** Verwendung eines Celluloseethers nach den Ansprüchen 1-4, **dadurch gekennzeichnet, dass** die quaternäre Ammoniumgruppe eine 2-Hydroxypropyltrialkylammoniumchloridgruppe ist, wobei die Alkylgruppen 1-22 Kohlenstoffatome enthalten.

**6.** Verwendung eines Celluloseethers nach den Ansprüchen 1-5, **dadurch gekennzeichnet, dass** die quaternäre Ammoniumgruppe eine 2-Hydroxypropyltrialkylammoniumchloridgruppe ist, wobei die Alkylgruppen 1-4 Kohlenstoffatome enthalten.

**7.** Verwendung eines Celluloseethers nach den Ansprüchen 1-6, **dadurch gekennzeichnet, dass** die quaternäre Ammoniumgruppe eine 2-Hydroxypropyltrimethylammoniumchloridgruppe ist.

**8.** Verwendung eines Celluloseethers nach den Ansprüchen 1-7, **dadurch gekennzeichnet, dass** er Substituenten

der Formel

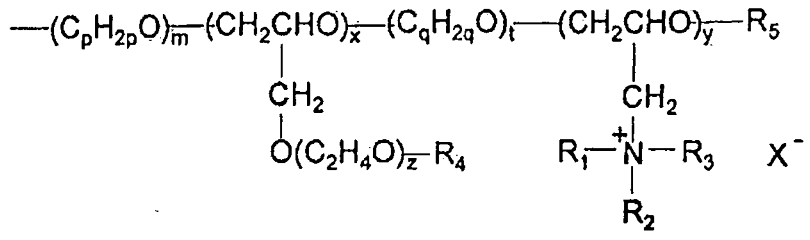

(I)

umfasst, wobei p und q unabhängig 2 oder 3 sind, m eine Zahl zwischen 0 und 5 ist, x eine Zahl zwischen 0 und 2 ist, t eine Zahl zwischen 0 und 5 ist, y eine Zahl zwischen 0 und 2 ist, z eine Zahl zwischen 0 und 10 ist, $R_1$, $R_2$ und $R_3$ unabhängig voneinander eine Alkylgruppe mit 1 - 22 Kohlenstoffatomen sind, $R_4$ eine Kohlenwasserstoffgruppe mit 5 - 22 Kohlenstoffatomen ist, $R_5$ eine Alkylgruppe mit 1 - 4 Kohlenstoffatomen oder H ist und X- ein Anion ist.

**9.** Verwendung eines Celluloseethers nach Anspruch 8, **dadurch gekennzeichnet, dass** x = 0 ist.

**10.** Verwendung eines Celluloseethers nach Anspruch 8, **dadurch gekennzeichnet, dass** er eine MS von 0,001 - 0,2 für die Gruppe

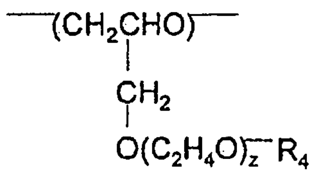

hat, wobei $R_4$ und z dieselbe Bedeutung wie in Formel (I) haben.

**11.** Verwendung eines Celluloseethers nach den Ansprüchen 1 - 10, **dadurch gekennzeichnet, dass** $R_5$ teilweise Methyl ist.

**12.** Verwendung eines Celluloseethers nach den Ansprüchen 1 - 11, wobei $R_1$, $R_2$ und $R_3$ unabhängig voneinander eine Alkylgruppe mit 1 - 4 Kohlenstoffatomen sind.

**13.** Verwendung eines Celluloseethers nach den Ansprüchen 1 - 12, **dadurch gekennzeichnet, dass** p und q 2 sind.

**14.** Verwendung eines Celluloseethers nach den Ansprüchen 1 - 13, **dadurch gekennzeichnet, dass** $R_1$, $R_2$ und $R_3$ $-CH_3$ sind.

**15.** Quaternärer Ammoniumhydroxyethylcelluloseether, **dadurch gekennzeichnet, dass** der Celluloseether die in den Ansprüchen 1 - 14 definierte Struktur hat, mit der Maßgabe, dass der Celluloseether keine hydrophobe Gruppe enthält, die aus der aus einem Aryl, Alkyl, Alkenyl, Aralkyl und Mischungen davon bestehenden Gruppe ausgewählt ist, und wenigstens 10 Kohlenstoffatome hat, wobei die hydrophobe Gruppe nicht Teil einer quaternären Ammoniumgruppe ist.

**16.** Verfahren zur Herstellung eines quaternären Ammoniumalkylhydroxyethylcelluloseethers nach Anspruch 15, **dadurch gekennzeichnet, dass** der Celluloseether hergestellt wird, indem eine quaternäre Ammoniumverbindung mit einem Hydroxyethylcelluloseether, der einen Ethersubstituenten umfasst, der eine Alkylgruppe mit 2 - 4 Kohlenstoffatomen und einen $DS_{Alkyl}$ von 0,1 - 2,5 aufweist, in Gegenwart von Alkali umgesetzt wird.

**17.** Verfahren nach Anspruch 16, wobei die quaternäre Ammoniumverbindung ein 2,3-Epoxypropyltrialkylammoniumchlorid oder ein 3-Chlor-2-hydroxypropyltrialkylammoniumchlorid ist, wobei die Alkylgruppen 1 - 22 Kohlenstoffatome

enthalten.

**18.** Verfahren nach Anspruch 17, wobei die Alkylgruppen 1 - 4 Kohlenstoffatome enthalten.

**19.** Verfahren nach Anspruch 18, wobei die Alkylgruppen Methylgruppen sind.

**20.** Wässrige Haar- oder Hautkonditionierungszusammensetzung, **dadurch gekennzeichnet, dass** sie Folgendes enthält:

- einen in Anspruch 1-15 definierten quaternären Ammoniumalkylhydroxyethylcelluloseether,
- ein nichtionisches, ein anionisches oder ein amphoteres Tensid oder eine Mischung davon, und
- ein Verdickungsmittel.

**21.** Wässrige Zusammensetzung nach Anspruch 20, die in einem Konditionierungsshampoo oder einem Körperreinigungsmittel zu verwenden ist, enthaltend

1) das nichtionische, ein anionisches oder ein amphoteres Tensid oder eine Mischung davon in einer Gesamtmenge von 5 bis 30 %
2) 0,2 bis 5 % Verdickungsmittel und
3) 0,05 bis 2 % des quaternären Ammoniuimalkylhydroxyethylcelluloseethers,

wobei die Zusammensetzung einen pH-Wert zwischen 5,0 und 7,0 hat.

**22.** Wässrige Zusammensetzung nach Anspruch 20, die in einem Haarkonditionierer zu verwenden ist, enthaltend

1) 0,05 bis 2 % des quaternären Ammoniuimalkylhydroxyethylcelluloseethers,
2) 0,5 bis 5 % eines langkettigen Fettalkohols,
3) eine hautverträgliche Säure in einer Menge, die ausreichend ist, um einen pH-Wert zwischen 2,5 und 5,5 zu erhalten.

**23.** Feste Seifenstück-Zusammensetzung, **dadurch gekennzeichnet, dass** sie einen in Anspruch 1-15 definierten quaternären Ammoniuimalkylhydroxyethylcelluloseether und ein Syndet, eine natürliche oder eine Kombinationsseife enthält.

**24.** Feste Seifenstück-Zusammensetzung nach Anspruch 23, enthaltend

1) ein anionisches Tensid, bei dem es sich um ein synthetisches anionisches Tensid und/oder eine Seife handelt, oder ein amphoteres Tensid oder eine Mischung davon in einer Gesamtmenge von 40 - 90 %,
2) 10 bis 60 % eines Weichmachers,
3) 0,05 bis 2 % des quaternären Ammoniuimalkylhydroxyethylcelluloseethers.

## Revendications

**1.** Utilisation d'un éther d'hydroxyéthylcellulose d'ammonium quaternaire, **caractérisé en ce qu'**il comprend un substituant éther contenant un groupe alkyle ayant de 2 à 4 atomes de carbone ayant un $DS_{alkyle}$ de 0,1 à 2,5, comme conditionneur capillaire ou cutané.

**2.** Utilisation d'un éther de cellulose selon la revendication 1, **caractérisé en ce que** le groupe alkyle comprend un groupe éthyle ayant un $DS_{éthyle}$ de 0,3 à 2,5.

**3.** Utilisation d'un éther de cellulose selon les revendications 1 à 2, **caractérisé en ce que** l'éther de cellulose a une $MS_{hydroxyéthyle}$ de 0,4 à 4,0.

**4.** Utilisation d'un éther de cellulose selon les revendications 1 à 3, **caractérisé en ce qu'**il a une $MS_{ammonium\ quaternaire}$ de 0,02 à 2,0.

**5.** Utilisation d'un éther de cellulose selon les revendications 1 à 4, **caractérisé en ce que** le groupe ammonium

quaternaire est un groupe chlorure de 2-hydroxypropyltrialkylammonium, où les groupes alkyles contiennent de 1 à 22 atomes de carbone.

**6.** Utilisation d'un éther de cellulose selon les revendications 1 à 5, **caractérisé en ce que** le groupe ammonium quaternaire est un groupe chlorure de 2-hydroxypropyltrialkylammonium, où les groupes alkyles contiennent de 1 à 4 atomes de carbone.

**7.** Utilisation d'un éther de cellulose selon les revendications 1 à 6, **caractérisé en ce que** le groupe ammonium quaternaire est un groupe chlorure de 2-hydroxypropyltriméthylammonium.

**8.** Utilisation d'un éther de cellulose selon les revendications 1 à 7, **caractérisé en ce qu'**il comprend des substituants de formule

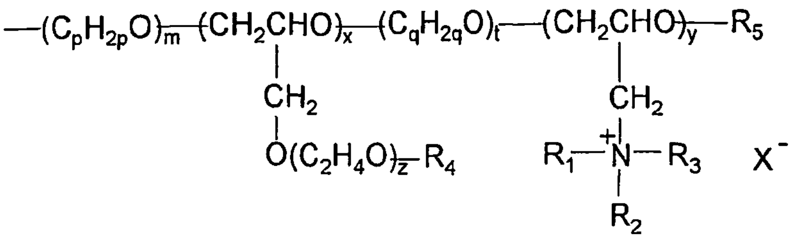

(I)

dans laquelle p et q, indépendamment, sont égaux à 2 ou 3, m est un nombre entre 0 et 5, x est un nombre entre 0 et 2, t est un nombre entre 0 et 5, y est un nombre entre 0 et 2, z est un nombre entre 0 et 10, $R_1$, $R_2$ et $R_3$, indépendamment les uns des autres, sont un groupe alkyle ayant de 1 à 22 atomes de carbone, $R_4$ est un groupe hydrocarbure ayant de 5 à 22 atomes de carbone, $R_5$ est un groupe alkyle ayant de 1 à 4 atomes de carbone ou H et $X^-$ est un anion.

**9.** Utilisation d'un éther de cellulose selon la revendication 8, **caractérisé en ce que** x=0.

**10.** Utilisation d'un éther de cellulose selon la revendication 8, **caractérisé en ce qu'**il a une MS de 0,001 à 0,2 pour le groupe

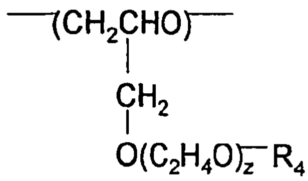

dans lequel $R_4$ et z ont la même signification que dans la formule (I).

**11.** Utilisation d'un éther de cellulose selon les revendications 1 à 10, **caractérisé en ce que** $R_5$ est partiellement méthyle.

**12.** Utilisation d'un éther de cellulose selon les revendications 1 à 11, dans lequel $R_1$, $R_2$ et $R_3$, indépendamment les uns des autres, sont un groupe alkyle ayant de 1 à 4 atomes de carbone.

**13.** Utilisation d'un éther de cellulose selon les revendications 1 à 12, **caractérisé en ce que** p et q sont égaux à 2.

**14.** Utilisation d'un éther de cellulose selon les revendications 1 à 13, **caractérisé en ce que** $R_1$, $R_2$ et $R_3$ sont $-CH_3$.

**15.** Ether d'hydroxyéthylcellulose d'ammonium quaternaire, **caractérisé en ce que** l'éther de cellulose a la structure définie dans les revendications 1 à 14, à la condition que l'éther de cellulose ne contienne pas de groupe hydrophobe

choisi dans le groupe constitué par aryle, alkyle, alcényle, aralkyle et leurs mélanges, et ayant au moins 10 atomes de carbone, le groupe hydrophobe ne faisant pas partie d'un groupe ammonium quaternaire.

**16.** Procédé de production d'un éther d'alkylhydroxyéthylcellulose d'ammonium quaternaire selon la revendication 15, **caractérisé en ce que** l'éther de cellulose est produit en faisant réagir un composé d'ammonium quaternaire avec un éther d'hydroxyéthylcellulose comprenant un substituant éther contenant un groupe alkyle ayant de 2 à 4 atomes de carbone et un $DS_{alkyle}$ de 0,1 à 2,5, en présence d'alcali.

**17.** Procédé selon la revendication 16, dans lequel le composé d'ammonium quaternaire est un chlorure de 2,3-époxypropyltrialkylammonium ou un chlorure de 3-chloro-2-hydroxypropyltrialkylammonium, où les groupes alkyles contiennent de 1 à 22 atomes de carbone.

**18.** Procédé selon la revendication 17, dans lequel les groupes alkyles contiennent de 1 à 4 atomes de carbone.

**19.** Procédé selon la revendication 18, dans lequel les groupes alkyles sont des groupes méthyles.

**20.** Composition aqueuse de conditionnement capillaire ou cutané, **caractérisée en ce qu'**elle contient

- un éther d'alkylhydroxyéthylcellulose d'ammonium quaternaire tel qu'il est défini dans les revendications 1 à 15,
- un tensioactif non ionique, anionique ou amphotère ou un mélange de ceux-ci, et
- un épaississant.

**21.** Composition aqueuse selon la revendication 20 à utiliser dans un shampooing conditionneur ou un produit nettoyant pour le corps contenant

1) le tensioactif non ionique, anionique ou amphotère ou un mélange de ceux-ci en une quantité totale de 5 à 30 %
2) 0,2 à 5 % d'épaississant, et
3) 0,05 à 2 % d'éther d'alkylhydroxyéthylcellulose d'ammonium quaternaire,

la composition ayant un pH entre 5,0 et 7,0.

**22.** Composition aqueuse selon la revendication 20 à utiliser dans un conditionneur capillaire contenant

1) 0,05 à 2 % de l'éther d'alkylhydroxyéthylcellulose d'ammonium quaternaire,
2) 0,5 à 5 % d'un alcool gras à longue chaîne
3) un acide compatible avec la peau en une quantité suffisante pour obtenir un pH entre 2,5 et 5,5.

**23.** Composition en pain de savon solide, **caractérisée en ce qu'**elle contient un éther d'alkylhydroxyéthylcellulose d'ammonium quaternaire tel qu'il est défini dans les revendications 1 à 15 et un savon syndet, naturel ou combiné.

**24.** Composition en pain de savon solide selon la revendication 23, contenant

1) un tensioactif anionique, qui est un tensioactif anionique synthétique et/ou un savon, ou un tensioactif amphotère ou un mélange de ceux-ci en une quantité totale de 40 à 90 %
2) 10 à 60 % d'un plastifiant
3) 0,05 à 2 % de l'éther d'alkylhydroxyéthylcellulose d'ammonium quaternaire.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 3472840 A
- WO 0006656 A
- US 3816616 A
- US 4228277 A
- EP 390240 A
- EP 426086 A
- WO 0008058 A


### Non-patent literature cited in the description


- **BERNARD IDSON.** *Cosmet. Sci. Technol. Ser.,* 1999, vol. 21, 251-279